# EUROPEAN PATENT APPLICATION

(11) **EP 3 964 192 A1**
(43) Date of publication of application: **09.03.2022**
(21) Application number: 20799316.3
(22) Date of filing: 28.04.2020
(51) Int. Cl.: A61K 8/365, A61Q 11/00

(54) **TOOTH WHITENING METHOD**

(30) Priority: 29.04.2019 JP 2019086987
(71) Applicant: Nishio, Hidetoshi, Chiba-shi, Chiba 260-0012 (JP)
(72) Inventor: Nishio, Hidetoshi, Chiba-shi, Chiba 260-0012 (JP)
(74) Representative: Wittmann, Günther
(86) International application number: PCT/JP2020/018101
(87) International publication number: WO 2020/222306

(57) **Abstract**

The purpose of the present invention is to provide a method such as polishing the tooth under suitable conditions to enhance whitening effects.

The following steps 1 to 3 are performed in number order, using a tooth polishing portion made of resin or resin composition having uneven surface, and citric acid or citric acid metal salts dispersed or dissolved in a gelatinous medium:
Step 1: Apply a gel of 1 to73% citric acid or citric acid metal salts to the tooth surface.
Step 2: Heat the tooth surface with the heating plate at 37°C-45°C for at least 3 minutes.
Step 3: Scrub the tooth surface with the tooth polishing portion to provide friction while removing the gel.

## Description

### [Technical Field]

The present invention relates to a tooth whitening method, and more particularly, to tooth whitening method using a tooth polishing tool.

### [Background Art]

Conventional tooth whitening involves demineralization of the tooth surface by using hydrogen peroxide to dissolve enamel proteins and whiten them (Patent Document 1).

Regardless of the method for whitening, it is effective to polish the tooth during tooth whitening. So, a melamine foam, which is a foam made of melamine resin, has been considered useful as a tooth polishing tool to remove yellowing or stains adhering to the tooth surface relatively easily, since it can effectively remove dirt on various objects due to its fine and uneven surface.

As a tooth whitening tool, a tooth polishing tool is described (Patent Document 2), wherein the tooth polishing tool comprising a handle, a support portion extending from the handle and a tool polishing portion attached to the support portion, and the polishing portion made of compressed melamine foam with a ratio of 1/3 to 1/5 between the number of septa on the surface parallel to the polished surface and the number of septa on the surface perpendicular to the polished surface.

However, the patent document 1 does not mention degradation of colored substances adhering near the tooth surface when whitening the tooth through demineralization of the tooth surface. The patent document 2 neither discusses agents to be used with the tooth polishing portion or a whitening method in detail, but only mentions that a tooth polishing portion made of melamine foam was immersed in water sufficiently as a condition for tooth whitening.

### [RELATED ART DOCUMENTS]

### [PATENT DOCUMENTS]

[Patent Document 1] JP2014-152166A
[Patent Document 2] JP2007-202809A

### [Disclosure of the Invention]

### [Problem to Be Solved by the Invention]

As mentioned above, even the use of highly abrasive tooth polishing portion has not yet produced enough whitening effects to meet users' demands. Therefore, there has been a need to develop a method that can increase the effectiveness of tooth whitening.

### [Means of Solving the Problems]

In response to the above issue, the inventor of the present invention has developed a tooth whitening method using a tooth polishing portion made of resin or resin composition having uneven surface, and citric acid. By contacting the citric acid dispersed or dissolved in a gel with colored substances between the enamel rods in an activated state for a certain period of time, the colored substances between the enamel rods that cannot be removed normally can be removed. As a result, the tooth whitening effect, especially the effect of decreasing the degree of staining and improving the whiteness, can be obtained. The tooth whitening method in the present invention includes at least the following (1) to (3):
(1) The tooth whitening method is to perform the following steps 1 to 3 in number order, using a tooth polishing portion made of resin or resin composition having uneven surface, and citric acid or citric acid metal salts dispersed or dissolved in a gelatinous medium.
   Step 1: Apply a gel of 1% to 73% citric acid or citric acid metal salts to the tooth surface.
   Step 2: Heat the tooth surface with the heating plate at 37°C-45°C for at least 3 minutes.
   Step 3: Rub the tooth surface with the tooth polishing portion to provide friction while removing the gel.
(2) The tooth whitening method according to (1), wherein apatite-containing agents are dispersed or dissolved in the gelatinous medium.
(3) The tooth whitening method according to (1) and (2), wherein the tooth polishing portion contains a melamine foam.

### [Effects of the Invention]

The tooth whitening method in the present invention enables to perform whitening with natural color of the teeth by removing the colored substances. At the same time, the tooth whitening effect with a higher degree of whiteness can also be obtained by selecting the agents to be used and adjusting the conditions, further increasing the tooth whitening effect.

The detailed mechanism of this effect is inferred as follows.

The colored substances between the enamel rods are bound or mixed with proteins and fixed to the tooth. The proteins are bound with calcium ion.

Chelating agents such as citric acid and its ions are known to improve the degradability of certain proteins, such as the inhibition of clotting. Also, in tooth whitening, it can chelate the calcium ion contained in a type of proteins, which are colored substances, to increase its solubility. This can make it harder for proteins to be fixed and easier for colored substances to be removed.

In addition, since the tooth whitening method in the present invention is different from the conventional tooth whitening method using hydrogen peroxide which involves demineralization of the tooth surface, the conventional tooth whitening method can be performed after removing colored substances by the tooth whitening method in the present invention. Therefore, when the whitening method in the present invention is used as a pre-treatment for the conventional whitening method, the tooth whitening effect can be further increased because it can prevent the contamination of colored substances into the tooth surface components which is formed by remineralization after demineralization.

In other words, in the whitening method in the present invention, the whitening effect can be achieved by the whitening method that uses a different action from the conventional one, or by the whitening method that uses a conventional action in addition to the aforementioned action.

### [Brief Description of Drawings]

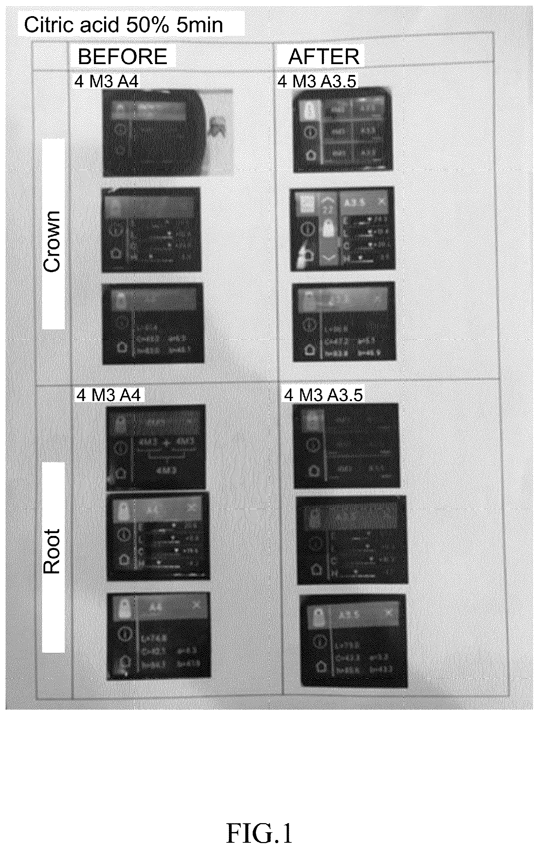

FIG.1 shows the results of the measuring the degree of staining of the crown and root of the tooth using the whitening effect measuring apparatus.

### [Mode for Carrying out the Invention]

Embodiments of the whitening method in the present invention will now be described below. However, the embodiments of the whitening method in the present invention are not limited to the followings.

### 1. First Embodiment

The first embodiment, one of the embodiments of the present invention, is the tooth whitening method using a tooth polishing portion made of resin or resin composition having uneven surface, citric acid or citric acid metal salts mixed or dissolved in a gelatinous medium. The method performs the following treatments in order: applying the gelatinous medium to the tooth, heating the applied gel and the surface of the tooth, and removing the gel after keeping the heated state for a certain period of time.

### <Resin or resin composition having uneven surface>

In the whitening method in the present invention, resin or resin composition having uneven surface is used to polish the tooth.

Preferably, the resin is a melamine foam, which is a foam made of melamine resin, such as, for example, urethane resin, melamine resin, polyester resin, acrylic resin, etc.

The unevenness of the resin may be formed by cross-sections of small holes, by another materials dispersed in the resin, or by molding the resin surface. In terms of the fact there is no significant change in the state of unevenness due to surface wear, it is preferable that the small holes that existed on the cut surface are formed by being broken up in cross-section.

### <Chelating agents>

The whitening method in the present invention preferably uses citric acid or citric acid salts as chelating agents. Furthermore, citric acid or sodium citrate salt are particularly preferred. However, to the extent that it does not interfere with the purpose of the present invention, it is not limited to citric acid, and other chelating agents may be used instead of or in combination with citric acid.

One example of the chelating agents is citric acid or the like, where the ligand group is a carboxyl group.

Chelating agents that are harmful to the human body, such as poisons and carcinogens, should be avoided. When using chelating agents that are or may be harmful to the human body, appropriate measures should be taken to remove them and prevent them from entering the body.

In the whitening method in the present invention, citric acid is used to chelate calcium ion. Citric acid has an inherent inhibitory effect on clotting. Proteins and other substances that are components of stained teeth also contain calcium, and chelation of calcium ions is thought to be effective in removing colored substances.

The whitening method in the present invention is used to remove colored substances between the enamel rods that cannot be removed normally by contacting the citric acid dispersed or dissolved in the gel with the colored substances between the enamel rods in an activated state for a certain period of time.

Citric acid may be a salt such as metal salts. In the present invention, citric acid is used either dispersed as particles in the gel or dissolved.

Citric acid and citric salts such as sodium citrate can break the linkage of colored substances on the tooth surface, due to their properties.

Also, the activity level of the citric ions can be increased by heating citric acid or citric salts at 37°C-45°C while they are applied to the tooth.

### <Gel>

The gel used in the whitening method in the present invention is not limited to those that can be applied to the teeth in the mouth. For example, the gel disclosed in the patent publication JP2016-104777A can be used.

In the present invention, the gel may dissolve the tooth whitening agents, or may be mixed with it to disperse the whitening agents in the medium.

The gel used in the whitening method in present invention includes, for example, polyvinylpyrrolidone, ethylene oxide, propylene oxide, and their copolymers. The gel used for the present invention may also include thickening agent to adjust viscosity. Any oral acceptable thickening agent can be used without restriction: carbomer, also known as carboxyvinyl polymer, or carrageenan, also known as Irish moss, and a more special type of carrageenan (Iota-carrageenan), cellulosic polymers such as high molecular weight polyethylene glycol, hydroxyethyl cellulose, carboxymethyl cellulose (CMC), and their salts, for example, sodium CMC, natural rubbers such as karaya, xanthan, gum arabic and tragacanth, colloidal magnesium aluminum silicate, and colloidal and/or fumed silica and mixtures thereof.

In various preferred embodiments in the whitening method in the present invention, a carrier is contained in the gel and citric acid or its chelating agents and other ingredients are dissolve or disperse in the gel. The carrier includes polymers and/or copolymers of polyethylene glycol, ethylene oxide/propylene oxide, and silicone. If those copolymers and/or polymers are used, they can be selected from commercially available substances. Block copolymers of ethylene oxide/propylene oxide are effective, but those with larger molecular weights, e.g., 5000 Da or more, are preferred. Low or medium molecular weight polyethylene glycols, e.g., PEG 400, PEG 600, PEG 800, PEG 1000, and mixtures thereof are also effective. It is preferred that the carrier impart a viscosity of from about 10,000 CPS to about 700,000CPS, preferably from about 30,000 CPS to about 300,000 CPS to the tooth polishing agents.

### <Tooth polishing portion>

The tooth polishing portion in the present invention is made of resin or resin composition.

The tooth polishing portion can be in of any shape as long as it does not interfere with the purpose of the present invention.

The tooth polishing portion has an uneven surface. The unevenness may be formed by cross-sections of small holes. In this case, the small holes are separated by septa of resin or resin composition, and the convex portion of the surface of the tooth polishing portion is the cross-sections of the septa.

The tooth polishing portion used for the present invention can be, for example, urethane resin, urea resin, nylon, silicone, acrylic resin, PVA resin, leather, etc., or non-woven fabric made of hemp, sisal hemp, felt cloth or polyester yarn, etc. In particular, thermosetting resin of a melamine foam or an urethane foam is preferred for the tooth polishing portion in the present invention.

### <Melamine foam and urethane foam>

In the whitening method of the present invention, the resin or resin composition having an uneven surface may contain a melamine foam. A melamine foam can be used as a tooth polishing portion (tooth polisher) and has a high polishing ability because it can polish the surface of the teeth while grinding it into a thin layer.

There are no particular restrictions on the melamine foam in the present invention to the extent that it does not interfere with the purpose of the present invention. Any material that is used in a wide variety of applications as a sweeping or polishing portion can be used as long as the conditions of hardness, elasticity, number and size of porous pores are met.

A compressed melamine foam may be used, which is made by heating and compressing a melamine foam in a foam where the cavity is separated by septa, while applying compressive force in the direction of its thickness. For example, it can be the melamine foam according to the method described in the patent publications, JP2002-172638 and JP2002-201299. In the compressed melamine foam, the surface with fine unevenness parallel to the compressed surface of the melamine foam is used as a tooth polishing surface.

Also, the septa on the surface (side surface) orthogonal to the polishing surface is as thick as the septa on the polishing surface.

Melamine foams that are commercially available as sweeping portions can be used as well. Depending on the conditions under which the whitening is to be performed, such as the size of citric acid or citrate particles, melamine foams with even higher number of small holes than those of common commercial products, such as those with 300-350/25mm of small pores, can also be used.

### <Tooth polishing tool>

There are no particular restrictions on the tooth polishing tool as long as it is capable of heating the gelatinous whitening agents and removing it after whitening.

The tooth polishing tools with high safety features, such as avoiding burns caused by overheating, are preferred.

The tooth polishing tool removes the colored substances in the enamel microcolumn sheath between enamel rods by polishing with the tooth polishing portion and heating.

When the colored substances are organic polymers (proteins), it can inhibit coagulation by chelating calcium ions that are necessary for coagulation.

### 2. Second Embodiment

The second embodiment, one of the embodiments of the present invention, is the tooth whitening method performing the following treatments in order: applying a tooth polishing portion made of resin or resin composition having uneven surface and citric acid or citric acid metal salts mixed or dissolved in a gelatinous medium to the tooth as gel medium, and heating the applied gel and the surface of the tooth, and removing the gel after keeping the heated state for a certain period of time, and then, applying a chemical containing hydrogen peroxide to the tooth surface, and remineralizing the tooth using apatite-containing agents.

The whiteness of the remineralized tooth surface can be further enhanced by removing proteins, which are colored substance, dispersed in the enamel rods sheath or the surfaces of the enamel rods using citric acid and by preventing colored substances from being incorporated into the remineralizing layer when remineralizing the tooth surface after the demineralization.

### < Apatite-containing agents >

In the whitening method in the present invention, the apatite-containing agents may be mixed or dissolved in the gelatious medium. The remineralizing effects of apatite can further enhance the whitening effect.

Any known apatite can be used as long as it does not interfere with the purpose of the present invention.

The apatite used for the whitening method in the present invention may be, for example, natural or artificial hydroxyapatite, etc. Having the remineralizing effects, it can repair the tooth surface and enhance the whiteness. To further whiten the tooth surface, an appropriate amount of pigments, dyes, etc. with whitening properties and less harmful effects, such as titanium dioxide, may be added to the apatite-containing agents.

### 3. Third Embodiment

The third embodiment, another variation of the embodiments of the present invention, is the whitening method that includes dispersing citric acid or citrate in the gel as fine particles smaller than the distance between the enamel rods.

The citric acid or citrate is not dissolved in the ingredients presenting in the enamel rods sheath, but rather the citric acid or citrate, which are active ingredients, are physically pushed into the pores of the enamel rods, further accelerating the breakdown of proteins. In addition, the fine particles with moderate hardness enhance the polishing effect on the tooth surface.

The size of the fine particles of the citric acid or citrate should be smaller than 0.1µm, which is smaller than the distance between the general enamel rods, and is as large enough to be pushed by the tooth polishing portion. Preferably, it is less than 0.05pm, more preferably, less than 0.025pm.

### [Example 1]

The following is one example of the present invention. The present invention is not limited to the following example.

### <Example>

A 50% citric acid gel was applied to the tooth surface including the root and crown areas. Next, the area where the gel was applied was heated for 5 minutes with the heating plate at 40°C. Then, a melamine foam was used to provide friction to the area while removing the citric acid gel.

When it was measured by an inspection device for measuring colorability (whitening measuring device "Shade Up Navi (SSN-1)" made by SHOFU INC.) after the citric acid is wiped off the tooth surface using the melamine foam, it was confirmed that the colored substances were removed and the degree of staining was reduced. FIG.1 shows the results showing that the degree of staining of the crown and root was decreased from A4 to A3.5 after the whitening.

It is therefore proved that the tooth whitening method in the present invention can reduce the degree of tooth staining and obtain whitening effects. More specifically, as shown in FIG.1, it was demonstrated that the degree of staining decreased in both the crown and root with the whitening method of the present invention, visually enhancing the whiteness of the tooth.

### [Industrial applicability]

The whitening method in the present invention can be used in cosmetic dentistry clinics, etc., as it can effectively whiten the tooth and make it look beautiful.

## Claims

1. The tooth whitening method which performs the following steps 1 to 3 in number order,
using a tooth polishing portion made of resin or resin composition having uneven surface,
and citric acid or citric acid metal salts dispersed or dissolved in a gelatinous medium:
Step 1: Apply the gel of 1 to73% citric acid or citric acid metal salts to the tooth surface.
Step 2: Heat the tooth surface with the heating plate at 37°C-45°C for at least 3 minutes.
Step 3: Provide friction to the tooth surface with the tooth polishing portion while removing the gel.

2. The tooth whitening method according to claim 1, wherein apatite-containing agents are dispersed or dissolved in the gelatinous medium.

3. The tooth whitening method according to claim 1 or claim 2, wherein the tooth polishing portion includes a melamine foam.
